# EUROPÄISCHE PATENTSCHRIFT

(11) **EP 3 600 195 B1**
(45) Veröffentlichungstag und Bekanntmachung des Hinweises auf die Patenterteilung: **14.10.2026**
(21) Anmeldenummer: 18717212.7
(22) Anmeldetag: 23.03.2018
(51) Int. Cl.: A61F 13/49, A61F 13/496, A61F 13/20, A61F 13/505, A41B 9/12, A61F 13/74

(54) **HOSE**
UNDERPANTS
SLIP

(30) Priorität: 27.03.2017 DE 102017002942; 12.07.2017 US 201762531629 P; 27.07.2017 DE 102017007014
(43) Veröffentlichungstag der Anmeldung: 05.02.2020
(73) Patentinhaber: Cuevas Dumois, Vivian, Barbara, 4000 Liège (BE)
(72) Erfinder: Cuevas Dumois, Vivian, Barbara, 4000 Liège (BE)
(74) Vertreter: Castell, Klaus
(86) Internationale Anmeldenummer: PCT/EP2018/000110
(87) Internationale Veröffentlichungsnummer: WO 2018/177586

(56) Entgegenhaltungen:
- CN-U- 202 207 244
- DE-A1- 102005 044 687
- DE-U1- 20 302 153
- DE-U1- 202004 019 377
- DE-U1- 202015 103 653
- US-A- 4 581 772

## Beschreibung

Eine Hose, die ein Vorderteil und ein Hinterteil aufweist, die im Schrittbereich miteinander verbunden sind, und die ein saugfähiges Vlies aufweist, wobei das Vorderteil und das Hinterteil im Taillenbereich an zwei gegenüberliegenden Seiten wie eine Windel lösbar miteinander verbunden sind, wobei das saugfähige Vlies mit der Hose fest verbunden ist und in die Hose eingearbeitet ist, ist aus der DE 10 2005 044 687 A1 und der DE 20 2015 103 653 U1 bekannt. Sie dient dazu, in extremen Einsätzen möglichst viel Flüssigkeit aufzunehmen.

Die Erfindung betrifft einen Damenslip gemäß Anspruch 1. Vorteilhafte Weiterbildungen sind Gegenstand der Unteransprüche.

In einen Damenslip kann eine Binde eingelegt werden. Als Alternative sind auch Damenslips bekannt, die eine saugfähige Schicht aufweisen.

In der Regel ist in einem Damenslip ein Einnäher vorgesehen, der auch Zwickel genannt wird. Der Zwickel sieht meist wie ein kleines Täschchen aus. Dieses Stück Stoff im Slip ist aber meist keine Tasche, sondern hat mehrere Funktionen.

Zwickel werden im Schrittbereich aus folgenden Gründen eingesetzt, wobei sich mehrere ergänzen oder auch ausschließen können:

Für eine bessere Hygiene ist ein Zwickel als zusätzliche teils ungefärbte Stofflage oder Plattierung im Intimbereich auf der Innenseite von Damenunterhosen weit verbreitet. Als Bezeichnungen sind die Worte Hygienezwickel, Schrittfutter, Beleg, Wäschezwickel oder einfach nur Zwickel gängig. Ein Zwickel kann auch eine Kombination aus dem Hauptmaterial, wie z.B. oft verwendete Baumwolle, als Baumwollzwickel sein. Auch werden Seide, Satin oder Microfaser verwendet. Teilweise sind diese noch mit einer antibakteriellen Wirkung versehen und dafür gedacht, Ausscheidungen, wie z. B. normalen Scheidenausfluss oder Schweiß aufzunehmen und durch Verdunstung trocknen zu lassen und so angenehmer auf der Haut im Intimbereich zu sein. So sollen auch Hautreizungen, Pilz- und Harnwegs-Infektionen reduziert werden. Das Obermaterial darüber kann auch ein Netzgewebe sein, um die Atmungsaktivität zu verbessern, welche bei einem Obermaterial aus Synthetikfaser bemängelt wird. Im Deutschen wird auch vom hygienischen Zwickel in Netzoptik gesprochen. Im Englischen spezifischer vom "Ventilated cotton gusset", "belüfteter Baumwollzwickel". Als kostengünstige Alternative auch nur als pures Netz, welches selbst oft nur aus Kunstfaser besteht. Auch bei der Verwendung von hydrophilen Stoffen wird hier die Feuchtigkeitsaufnahme als unzureichend bemängelt. Gewebestärken reichen von zwei Stofflagen bis Netzstruktur. Die Schamspalte wird bei Unterwäsche meist nicht vollständig bedeckt. Bei ungenügender Funktion bzw. Fehlen dieses Zwickels kann eine Slipeinlage eingeklebt oder zusätzliche Unterwäsche getragen werden. Unterwäsche ohne zweite Stofflage oder Plattierung kann auch dicker gearbeitet oder mit Wabenstrukturen versehen sein.

Ein Einnäher im Slip schützt vor dem rauem Stoff der Unterwäsche und auch vor anderen äußeren Einwirkungen, wie etwa Jeans.

Außerdem verbessert es bei Damen die Scheidenflora, da es den Ausfluss aufsaugt und ihn schneller trocknen lässt. Daher ist der Zwickel meist aus Baumwolle, weil diese besonders saugfähig ist. So entsteht kein zu feuchtes Klima, das Bakterienbildung fördern könnte.

Die Hose ist im vorliegenden Fall ein Damenslip. Dieser Damenslip ist aus textilem Material in üblicher Form eines Damenslips hergestellt, wobei nur im Schrittbereich eine Einlage vorgesehen ist, die als saugfähiges Vlies sich nur auf den Schrittbereich begrenzt. Im Gegensatz zu einer Windel bedeckt die erfindungsgemäße Einlage nur den Schrittbereich und insbesondere nur maximal 50 % und vorzugsweise weniger als 30 % der Oberfläche der Hose. Bei einem Tanga kann im Wesentlichen nur das saugfähige Vlies mit schmalen Befestigungsbändern die Hose bilden.

Gerade wenn die Hose oder der Slip nur aus dem Zwickel und Bändern oder schmalen Stoffstreifen besteht, liegt die Funktion der Hose vor allem in der Funktion des Zwickels, der durch den Rest der Hose nur gehalten wird und ggf. mit Spitzen etc. verschönert wird.

Wenn der Zwickel mit einem saugfähigen Material gefüllt ist, dann kann der Zwickel im nicht gepressten Zustand eine Dicke von mehr als 0,5 cm, vorzugsweise mehr als 1 cm oder sogar mehr als 1,5 cm aufweisen. Dies ermöglicht es, sehr viel saugfähiges Material im Zwickel anzuordnen. Insbesondere in einem solchen Fall ist es vorteilhaft, wenn der Zwickel eine gebogene Oberfläche mit im mittleren Bereich zwischen den Beinöffnungen einer Erhöhung aufweist, die durch die stärkere Dicke in diesem Bereich besonders saugfähig ist. Außerdem kann dieser Bereich konvex gestaltet sein, sodass er mit einer konkaven Oberfläche des Körpers des Hosennutzers beim Tragen der Hose zusammenwirken kann. Dies kann im einfachsten Fall eine regionale Erhöhung sein, es kann eine sich in der Längserstreckung des Zwickels erstreckende Wulst sein oder sogar eine tamponartig ausgebildete Erhöhung.

Diese Einlage wird vorzugsweise zusammen mit der Hose gefertigt und einstückig mit der Hose vertrieben. Bei einer Verschmutzung der Einlage muss somit die saugfähige Vlieseinlage zusammen mit der textilen Hose entsorgt oder gewaschen werden.

Dafür kann der Slip an den beiden Seiten geöffnet werden und beispielsweise ohne eine Überhose ausziehen zu müssen, ausgezogen werden, indem der Slip zwischen den Beinen herausgezogen wird.

Fest verbunden heißt, dass das saugfähige Vlies nicht lose in der Hose liegt sondern nicht verschiebbar mit der Hose verbunden ist. Das ist im Extremfall eine Hose mit einem einstückig eingearbeiteten Vlies. Das kann aber auch ein in die Hose eingenähtes Vlies sein. Das kann ein in eine Tasche in der Hose eingelegtes und aus der Tasche auch wieder herausnehmbares Vlies sein oder ein Vlies, das beispielsweise über einen Klettverschluss fest mit der Hose verbunden ist. In allen Fällen wurde somit die Hose für das Vlies besonders hergerichtet.

Zum Öffnen können seitlich am Slip auf gegenüberliegenden Seiten beispielsweise Klettverschlüsse, eine Druckknöpfe, Haken und Ösen oder Hosenknöpfe vorgesehen sein.

Dabei kann der Slip auch als Spitzenslip oder Tangaslip ausgebildet sein.

Dies ermöglicht es, die Hose zu wechseln ohne eine Überhose oder die Schuhe ausziehen zu müssen. Somit kann in einer passenden kleinen Tasche eine neue Hose bzw. ein neuer kleiner Slip mitgeführt werden, der leicht ausgepackt und angezogen werden kann. Die getragene Hose dann in die kleine Tasche gelegt werden und später entsorgt oder gewaschen werden.

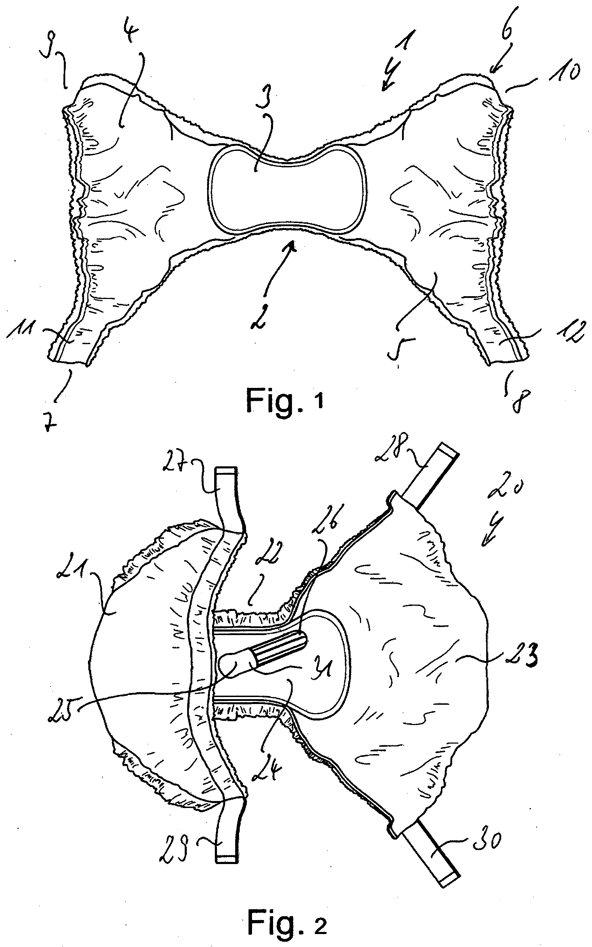

Besonders vorteilhaft ist es, wenn die Einlage der Hose auch ein am saugfähigen Vlies zum Vlies beweglich befestigtes saugfähiges Element wie etwa einen Tampon aufweist, der an der Einlage befestigt ist. Ein vorteilhaftes Ausführungsbeispiel ohne Tampon ist in Figur 1 dargestellt und ein Ausführungsbeispiel mit Tampon zeigt die Figur 2.

Vorteilhaft ist es, wenn das saugfähige Vlies Flüssigkeitsleitadern aufweist. Diese können sich wie Adern eines Blattes eines Laubbaumes fächerförmig (Ginko oder Orchidee) erstrecken oder auch verzweigen (Ahorn).

Diese Adern ermöglichen es, die Flüssigkeit vom Tampon weg zu transportieren. Sie können auch Flüssigkeit an das Vlies abgeben, um einen größeren Bereich des Vlieses oder sogar das gesamte Vlies zu verwenden, um Flüssigkeit aufzunehmen. Dafür kann das Vlies Superabsorber oder andere flüssigkeitsbindende Substanzen aufweisen.

Die Verteilung der Flüssigkeit hilft auch dabei, eine Kontamination von Blut schnell zu erkennen.

Die Hose oder zumindest das Vlies sind als Einwegprodukt konzipiert und vorzugsweise nur zum Gebrauch spätestens bis zur ersten Kontamination.

Vorteilhafte Weiterbildungen sind Gegenstand der Unteransprüche. Ein erfindungsgemäßes Ausführungsbeispiel ist in der Zeichnung dargestellt und wird im Folgenden näher beschreiben.

Er zeigt
- Figur 1: eine Draufsicht auf einen geöffneten Damenslip,
- Figur 2: einen geöffneten Damenslip mit Tampon und die
- Figuren 3 bis 5: weitere Ausführungsbeispiele.

Die Figur 1 zeigt eine Hose 1, insbesondere eine Unterhose oder einen Damenslip, die im Schrittbereich 2 ein saugfähiges Vlies 3 aufweist. Dieses saugfähige Vlies 3 ist mit der übrigen Hose 1 festverbunden und nach Art eines Zwickels in den Damenslip eingearbeitet. Diese als Damenslip ausgebildete Hose 1 hat ein Vorderteil 4 und ein Hinterteil 5, die im Schrittbereich 2 miteinander verbunden sind. Im Taillenbereich, in dem das Vorderteil 4 und das Hinterteil 5 an der Hüfte anliegen, hat die Hose an zwei gegenüberliegenden Seiten 7, 8 und 9, 10 Verschlüsse 11, 12 um mit dem Verschluss 12 die Seiten 7 und 8 miteinander zu verbinden und sprechend mit einem Verschluss (nicht gezeigt) die Seiten 9 und 10 miteinander zu verbinden.

Das saugfähige Vlies 3 ist dabei in die Hose 1 eingearbeitet. Die Hose kann auch die Form eines Tangas haben und sie ist im Ausführungsbeispiel als Einweghose ausgebildet, die zur Aufnahme von flüssigen Ausscheidungen (Menstruation, Scheidenausfluss, Schweiß etc.) geeignet.

Die Figur 2 zeigt ein Ausführungsbeispiel, das im Wesentlichen dem Ausführungsbeispiel gemäß Figur 1 entspricht. Hier sind bei einer Hose 20 ein Vorderteil 21 in Schrittbereich 22 mit einem Hinterteil 23 verbunden und das Vlies 24 ist über ein flexibles Verbindungsstück 25 mit einem ergonomischen Tampon 26 zur Aufnahme von flüssigen Ausscheidungen verbunden. Der Tampon 26 ist somit als saugfähiges Element flexibel mit dem saugfähigen Vlies 3 verbunden. Dies ermöglicht es, den Bereich des Vlieses 3 relativ zur Scheide der Dame, die die Hose 1 trägt, zu positionieren.

Das Vorderteil 21 wird einerseits über Verschlüsse 27 und 28 und andererseits über Verschlüsse 29 und 30 mit dem Hinterteil 23 verbunden. Das flexible Verbindungsstück 25 hat an seinem dem saugfähigen Vlies 3 abgewandten Ende eine Hülse 31, in die der Tampon 26 teilweise eingesteckt werden kann.

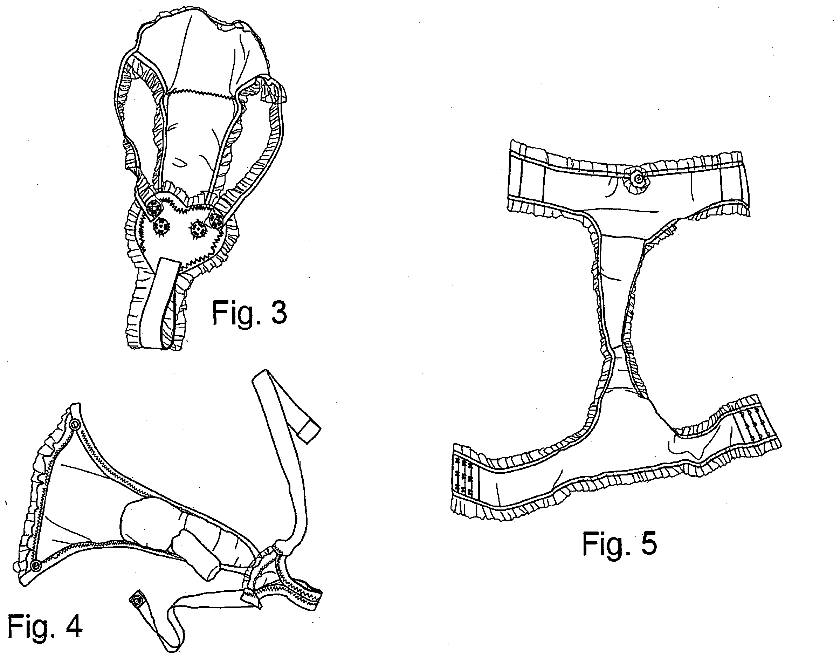

Die in den Figuren 3 bis 5 gezeigten weiteren Ausgestaltungen zeigen, wie das saugfähige Vlies im Schrittbereich auf einen im Wesentlichen spitzwinkligen, dreieckigen Bereich beschränkt werden kann, während die Hose auf unterschiedliche Art und Weise als Damenslip ausgebildet werden kann. Die Verbindung von Vorderteil und Hinterteil kann dabei ähnlich wie bei einem Büstenhalter mit parallelen Ösenleisten ausgebildet sein, in die eine Hakenleiste eingreift. Die Ausführungsbeispiele zeigen, dass unterschiedlichste Damenslips hergestellt werden können, die einerseits wenig Stofffläche bieten und andererseits im Schrittbereich einen saugfähigen Vliesbereich, der dazu ausgebildet ist, Flüssigkeiten aufzunehmen. Dieser Schrittbereich kann mit speziellen hochsaugfähigen Materialien, wie beispielsweise Superabsorbern bestückt sein, um die Saugfähigkeit dieses Bereiches zu optimieren, während die übrigen Bereiche des Damenslips optisch ansprechend in unterschiedlicher Form ausgebildet sein können.

Der Damenslip kann beispielsweise mehrere Lagen aufweisen, die dort angeordnet sind, wo man normalerweise die Binde einlegt. Ganz innen ist eine Lage vorgesehen, die für eine sich trocken anfühlende Oberfläche sorgt. Dann kommt eine antibakterielle Schicht, dann kommt eine absorbierende Schicht, die Flüssigkeit aufnehmen kann, und die äußerste Schicht ist eine abweisende Lage, die den Übergang von Flüssigkeit auf eine darüber getragene Hose verhindert. Der Slip ermöglicht es generell bei Inkontinenz oder bei einer Dame auch während ihrer Tage ohne Binde, Einlage oder Windel auszukommen. Das seitliche Öffnen der Hose ermöglicht ein schnelles Wechseln der Hose. Dadurch reicht ein kleinerer Bereich für das saugfähige Material aus, das auf den Zwickel der Hose begrenzt werden kann.

Eine V-Form des saugfähigen Vlieses ermöglicht eine besonders gute Anpassung an die weibliche Anatomie im Genitalbereich durch eine optimale Bedeckung des Genitalbereichs einschließlich der Vulva und sie wirkt einem Aufrollen des Vlieses durch Bewegung entgegen. Ein derartiges V-förmiges Vlies kann mit oder ohne Tampon ausgebildet sein. Das Vlies hat vorzugsweise eine Wattierung.

Für die Einmalverwendung kann die Hose als Einweghose ausgebildet sein. Sofern die Hose ein Tampon oder ein tamponähnliches Saugelement aufweist, kann dieses Poren für eine bessere Belüftung des Genitalbereichs aufweisen. Eine flexible Befestigung eines Tampons erlaubt eine automatische Anpassung an unterschiedliche Anatomien und sie sorgt für einen besseren Tragekomfort bei Bewegungen.

Das saugfähige Vlies kann auch eine ovale Form ausweisen.

Der Zwickel kann auch hantelförmig sein.

Die Ausscheidungen können mit einer derartigen Hose zentralisiert vom Vlies aufgenommen werden. Diese Hosen sind besonders für Frauen im menstruationsfähigen Alter geeignet.

## Patentansprüche

1. Hose (1), die ein Vorderteil (4) und ein Hinterteil (5) aufweist, die im Schrittbereich (2) miteinander verbunden sind, und die im Schrittbereich (2) ein saugfähiges Vlies (3) aufweist, wobei das Vorderteil (4) und das Hinterteil (5) im Taillenbereich (6) an zwei gegenüberliegenden Seiten (7, 9 und 8, 10) wie eine Windel lösbar miteinander verbunden sind, wobei das saugfähige Vlies (3) mit der Hose (1) fest verbunden ist und in die Hose (1) eingearbeitet ist, ***dadurch gekennzeichnet, dass*** die Hose (1) ein Damenslip ist, das saugfähige Vlies sich nur auf den Schrittbereich begrenzt und maximal 50 % der Oberfläche der Hose bedeckt.

2. Hose nach Anspruch 1, ***dadurch gekennzeichnet, dass*** das saugfähige Vlies (3) mit einem Tampon (26) verbunden ist.

3. Hose nach Anspruch 2, ***dadurch gekennzeichnet, dass*** die Hose ein flexibles Verbindungsteil (25) aufweist, das mit dem Vlies (3) und dem Tampon (26) verbunden ist.

4. Hose nach Anspruch 3, ***dadurch gekennzeichnet, dass*** das Verbindungsteil eine Hülse (31) aufweist, in die das Tampon (26) teilweise eingesteckt ist.

5. Hose (1) nach einem der vorhergehenden Ansprüche, ***dadurch gekennzeichnet, dass*** das saugfähige Vlies (3) eine mit der Hose (1) fest verbundene Inkontinenzeinlage ist.

6. Hose (1) nach einem der vorhergehenden Ansprüche, ***dadurch gekennzeichnet, dass*** das saugfähige Vlies (3) eine im Wesentlichen dreieckige Grundform aufweist.

7. Hose (1) nach einem der vorhergehenden Ansprüche, ***dadurch gekennzeichnet, dass*** das saugfähige Vlies (3) Flüssigkeitsleitadern aufweist.

8. Hose (1) nach Anspruch 7, ***dadurch gekennzeichnet, dass*** die Flüssigkeitsleitadern an einem Tampon (26) beginnen und sich in das Vlies (3) erstrecken.

9. Hose (1) nach Anspruch 7 oder 8, ***dadurch gekennzeichnet, dass*** sich die Flüssigkeitsleitadern wie Adern eines Blattes verzweigen und zu ihrem Ende hin verjüngen.

## Claims

1. Pants (1) comprising a front portion (4) and a rear portion (5), which are connected to one another in a crotch region (2), and comprising an absorbent nonwoven material (3) in the crotch region (2), wherein the front portion (4) and the rear portion (5) are releasably connected to one another in a waist region (6) at two opposite sides (7, 9 and 8, 10) in the manner of a diaper, wherein the absorbent nonwoven material (3) is permanently connected to and incorporated into the pants (1), ***characterized in that*** the pants (1) are women's briefs, the absorbent nonwoven material is limited only to the crotch region and covers at most 50% of the surface area of the pants.

2. Pants according to claim 1, ***characterized in that*** the absorbent nonwoven material (3) is connected to a tampon (26).

3. Pants according to claim 2, ***characterized in that*** the pants comprise a flexible connecting member (25) connected to the nonwoven material (3) and the tampon (26).

4. Pants according to claim 3, ***characterized in that*** the connecting member comprises a sleeve (31) into which the tampon (26) is partially inserted.

5. Pants (1) according to any one of the preceding claims, ***characterized in that*** the absorbent nonwoven material (3) is an incontinence pad permanently connected to the pants (1).

6. Pants (1) according to any one of the preceding claims, ***characterized in that*** the absorbent nonwoven material (3) has a substantially triangular basic shape.

7. Pants (1) according to any one of the preceding claims, ***characterized in that*** the absorbent nonwoven material (3) comprises liquid-conducting channels.

8. Pants (1) according to claim 7, ***characterized in that*** the liquid-conducting channels begin at a tampon (26) and extend into the nonwoven material (3).

9. Pants (1) according to claim 7 or 8, ***characterized in that*** the liquid-conducting channels branch out like the veins of a leaf and taper towards their ends.

## Revendications

1. Culotte (1) comprenant une partie avant (4) et une partie arrière (5), reliées l'une à l'autre dans une région d'entrejambe (2), et comprenant un insert non tissé absorbant (3) dans la région d'entrejambe (2), la partie avant (4) et la partie arrière (5) étant reliées de manière détachable l'une à l'autre dans une région de taille (6) sur deux côtés opposés (7, 9 ; 8, 10), à la manière d'une couche, l'insert non tissé absorbant (3) étant fixé de manière permanente à la culotte (1) et intégré dans celle-ci, ***caractérisée en ce que*** la culotte (1) est une culotte pour femme, l'insert non tissé absorbant étant limité exclusivement à la région d'entrejambe et couvrant au plus 50 % de la surface de la culotte.

2. Culotte selon la revendication 1, ***caractérisée en ce que*** l'insert non tissé absorbant (3) est relié à un tampon (26).

3. Culotte selon la revendication 2, ***caractérisée en ce que*** la culotte comprend un élément de liaison flexible (25) relié à l'insert non tissé (3) et au tampon (26).

4. Culotte selon la revendication 3, ***caractérisée en ce que*** l'élément de liaison comprend un manchon de réception (31) dans lequel le tampon (26) est partiellement introduit.

5. Culotte (1) selon l'une quelconque des revendications précédentes, ***caractérisée en ce que*** l'insert non tissé absorbant (3) constitue une protection contre l'incontinence fixée de manière permanente à la culotte (1).

6. Culotte (1) selon l'une quelconque des revendications précédentes, ***caractérisée en ce que*** l'insert non tissé absorbant (3) présente une forme sensiblement triangulaire.

7. Culotte (1) selon l'une quelconque des revendications précédentes, ***caractérisée en ce que*** l'insert non tissé absorbant (3) comprend des canaux de guidage de fluide.

8. Culotte (1) selon la revendication 7, ***caractérisée en ce que*** les canaux de guidage de fluide prennent naissance au niveau d'un tampon (26) et s'étendent dans l'insert non tissé (3).

9. Culotte (1) selon la revendication 7 ou 8, ***caractérisée en ce que*** les canaux de guidage de fluide se ramifient à la manière des nervures d'une feuille et s'amincissent vers leurs extrémités
